(19) **Europäisches Patentamt**
**European Patent Office**
**Office européen des brevets**

(11) **EP 1 106 596 B1**

(12) **EUROPÄISCHE PATENTSCHRIFT**

(45) Veröffentlichungstag und Bekanntmachung des Hinweises auf die Patenterteilung:
**05.03.2008  Patentblatt 2008/10**

(51) Int Cl.:
**B01J 8/06** *(2006.01)*       **C07C 45/74** *(2006.01)*
**C07C 47/21** *(2006.01)*

(21) Anmeldenummer: **00122424.5**

(22) Anmeldetag: **13.10.2000**

(54) **Verfahren zur katalytischen Durchführung von Aldolkonensationen mittels Mehrphasenreaktion**

Process to perform aldolcondensations using catalysed multiphase reactions

Procédé pour la mise en oeuvre de condensations aldoliques à l'aide de réactions catalysées multiphasiques

(84) Benannte Vertragsstaaten:
**AT BE CH CY DE DK ES FI FR GB GR IE IT LI LU MC NL PT SE**

(30) Priorität: **30.11.1999  DE 19957522**

(43) Veröffentlichungstag der Anmeldung:
**13.06.2001  Patentblatt 2001/24**

(73) Patentinhaber: **Evonik Oxeno GmbH**
**45772 Marl (DE)**

(72) Erfinder:
• **Wiese, Klaus-Diether, Dr.**
**45721 Haltern (DE)**
• **Protzmann, Guido, Dr.**
**45772 Marl (DE)**

• **Koch, Jürgen**
**45721 Haltern (DE)**
• **Büschken, Wilfried, Dr.**
**45721 Haltern (DE)**

(74) Vertreter: **Hirsch, Hans-Ludwig et al**
**Evonik Degussa GmbH**
**DG-IPM-PAT**
**Bau 1042 / PB 15**
**Paul-Baumann-Strasse 1**
**45764 Marl (DE)**

(56) Entgegenhaltungen:
**EP-A- 1 057 524**       **EP-A- 1 057 525**
**GB-A- 761 203**       **US-A- 3 077 500**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Durchführung von Mehrphasenreaktionen in einem Rohrreaktor, insbesondere zur Herstellung von $\alpha,\beta$-ungesättigten Aldehyden durch Aldolkondensation von Aldehyden.

Ungesättigte Aldehyde sind wegen ihrer Reaktivität Edukte für die Herstellung einer Vielzahl von organischen Verbindungen. Ihre Selektivhydrierung ergibt die entsprechenden gesättigten Aldehyde, die ebenfalls Basis vieler Synthesen sind. Die Oxidation der Aldehyde führt zu Carbonsäuren, die technisch genutzt werden. Die Hydrierung der Aldehyde führt zu gesättigten Alkoholen, die zur Herstellung von Weichmachern und Detergentien verwendet werden.

Die Aldolreaktion von n-Butyraldehyd unter gleichzeitiger Wasserabspaltung zu 2-Ethylhexenal wird weltweit in großem Maßstab ausgeübt, da das Hydrierprodukt, 2-Ethylhexanol, in großem Umfang als Weichmacheralkohol eingesetzt wird. Als Katalysator dient üblicherweise eine Base, gelöst in Wasser. Typisch ist die Verwendung von wäßriger Natronlauge mit einem Gehalt von NaOH im Prozentbereich. Die Reaktion wird häufig in einem Temperaturbereich von 80-150 °C, einem Druck unter 5 bar und einem Phasenverhältnis zwischen organischer Phase und Katalysatorphase von 1:20 durchgeführt (Hydrocarbon Processing, October 1980, Section 2, pages 93-102). Diese Reaktion kann z.B. in einem Rührkessel (DE 19 06 850, DE 927 626), in einer gepackten Säule, die im Gegenstrom betrieben wird (G. Dümbgen, D. Neubauer, Chemie-Ing.-Techn., 41, 974 (1969), oder in einem Strömungsrohr (GB 761 203) durchgeführt werden. Alle diese Verfahren liefern bei Umsätzen von 98,5 % 2-Ethylhexenal in einer Selektivität von bis zu 98 %. Nachteilig dabei ist, daß bei relativ hohen Temperaturen ein Teil des eingesetzten n-Butyraldehyds durch Cannizzaro-Reaktion irreversibel verloren geht. Die bei der Cannizzaro-Reaktion gebildete Buttersäure neutralisiert den basischen Katalysator. Es muß daher ständig ein Teil der Katalysatorlösung mit einer hohen Fracht an organischem Material ausgeschleust und durch frischen Katalysator ersetzt werden.

Analog läßt sich Valeraldehyd zu 2-Propyl-Heptenal umsetzen. Die Aldolkondensation der $C_5$-Aldehyde kann in gerührten Reaktoren durchgeführt werden, die zur Abführung der Wärme mit innenliegenden Wärmetauschern ausgerüstet sind. Diese Reaktionsführung wird z. B. in WO 93/20034 beschrieben und ist aufgrund der bewegten Teile mechanisch anfällig und in Bau und Instandhaltung wegen der in den Reaktor eingebauten Wärmetauscher aufwendig.

Bei Aldehyden mit mehr als 6 Kohlenstoffatomen ist die Reaktionsgeschwindigkeit wegen der geringen Löslichkeit der Aldehyde in der wäßrigen Katalysatorphase noch geringer und damit oft nicht mehr wirtschaftlich. Ebenfalls schwierig durchführbar wird die Aldolkondensation bei Verwendung verzweigter Aldehyde, wie z. B. 3-Methylbutanal.

Die Kondensation von Aldehyden kann auch in homogener Phase, z.B. mit Aminen als Katalysator, durchgeführt werden. Diese Verfahren weisen die Nachteile auf, daß mehr Nebenprodukte entstehen und der Katalysator aus dem Produktgemisch abgetrennt werden muß. Daher werden technische Aldolkondensationen vorzugsweise als Mehrphasenreaktionen, insbesondere als Zweiphasenreaktion, durchgeführt.

Unter Zweiphasenrektion werden im folgenden Reaktionen verstanden, die unter Beteiligung von zwei nicht oder nur teilweise mischbaren fluiden Phasen ablaufen. Bei der Aldolkondensation von Aldehyden liegen zwei Flüssigphasen vor, die nicht mischbar sind oder eine Mischungslücke aufweisen. Zu Beginn der Umsetzung bestehen die beiden Phasen aus Edukt und Katalysatorlösung, nach erfolgter Reaktion aus Produkt- und Katalysatorphase.

Bei jeder Zweiphasenreaktion ist das Problem des Stoffübergangs zu überwinden. Die Edukte müssen in die Katalysatorphase transportiert werden und die Produkte z. T. wieder zurück. Da Transportvorgänge häufig langsamer sind als die eigentliche Reaktion, sind solche Reaktionen durch die Geschwindigkeit des Stoffüberganges bestimmt, man spricht von einer transportgehemmten Reaktion.

Um bei einer Mehrphasenreaktion, insbesondere bei jenen, bei denen die Phasen kaum ineinander löslich sind, technisch akzeptable Raum-Zeit-Ausbeuten zu erhalten, müssen die Stoffe möglichst innig miteinander in Kontakt gebracht werden. Es muß eine möglichst große Stoffübergangsfläche $a_s$ zwischen den Phasen erzeugt werden. Andererseits müssen die Phasen nach erfolgter Reaktion wieder leicht getrennt werden können. Zu starke Vermischung kann hier zu Schwierigkeiten führen, da Emulsionen entstehen können.

Neben einer hohen Stoffübergangsfläche $a_s$ sollte in allen Mehrphasenreaktionen ein möglichst hoher Stoffübergangskoeffizient $k_l$ erreicht werden. Insgesamt sollte der sogenannte KLA-Wert, d.h. das Produkt aus $k_l$ und $a_s$ in der Stoffübergangsgleichung

$$j = k_l a_s (C^* - C)$$

mit

j    [Mol/s] der durch die Phasengrenzfläche hindurchtretende Molenstrom der reagierenden Komponente, (z. B. Eintritt von Aldehyd in die Katalysatorphase),

$k_l$    [m/s] Stoffubergangskoeffizient,

$a_s$     [m$^2$] Phasengrenzfläche im Reaktor, (z. B. Aldehyd in der Katalysatorphase),

C*     [Mol/m$^3$] maximale Löslichkeit des Edukts in der zweiten Phase und

C     [Mol/m$^3$] tatsächliche Konzentration des Edukts, die wiederum mit der Reaktionsgeschwindigkeit gekoppelt ist,

maximal sein.

Ein weiteres Problem bei Mehrphasenreaktionen ist die Wärmeabfuhr bei exothermen Reaktionen. Gelingt es, die Reaktionsgeschwindigkeit durch Verbesserung des Stoffüberganges zu erhöhen, muß naturgemäß auch mehr Wärme abgeführt werden, was zu unerwünschter Temperaturerhöhung bis hin zum Durchgehen der Reaktion führen kann.

Daher wird eine zweiphasige Aldolkondensation häufig im Rührkessel durchgeführt. Dabei nimmt man die ständige Rückvermischung in Kauf, wodurch die effektive Konzentration der Reaktanden herabgesetzt wird. Dies führt zur Absenkung der Raumzeitausbeute, was wiederum durch vergrößerten Reaktionsraum ausgeglichen werden muß.

Alternativ könnte die Zweiphasenreaktion auch in einem Strömungsrohr durchgeführt werden. Hier besteht jedoch die Gefahr, daß sich die Phasen entmischen und die Reaktionsgeschwindigkeit zu stark abnimmt. Weiterhin ist die bereits diskutierte Problematik der Wärmeabfuhr zu beachten.

Aufgabe der vorliegenden Erfindung ist somit die Bereitstellung eines Verfahrens zur Durchführung von Mehrphasenreaktionen, das sich besonders für die Durchführung von Aldolkondensationen eignet.

Technisch sollte das neue Verfahren folgende Ansprüche an ein Mehrphasenverfahren erfüllen:

- Erzeugung eines hohen und stabilen Stoffübergangs zwischen den beteiligten Phasen
- Einfach ausführbar, möglichst mit üblichen technischen Apparaten
- Einfache und sichere Wärmeabfuhr
- Hohe Betriebssicherheit
- Einfache und sichere Maßstabsübertragung

In Bezug auf die durchzuführende Herstellung von $\alpha,\beta$-ungesättigten Aldehyden durch Aldolkondensation kommen speziell hinzu:

- Hohe Selektivität, Vermeidung insbesondere hochsiedender Nebenprodukte
- Zurückdrängung der Cannizzaro-Reaktion, daher keine oder nur geringfügige Katalysatorausschleusung
- Hohe Raum-Zeit-Ausbeute, kleine Reaktoren
- Hohe Produktreinheit

Gegenstand der vorliegenden Erfindung ist daher ein Verfahren zur katalytischen Aldolkondensation von Aldehyden durch Mehrphasenreaktion in einem Rohrreaktor, wobei der Katalysator in der kontinuierlichen Phase und mindestens ein Aldehyd in einer dispergierten Phase enthalten ist und der Belastungsfaktor B des Reaktors gleich oder größer 0,8 ist.

Mit dem erfindungsgemäßen Verfahren wurde ein überraschend einfaches Verfahren zur Durchführung von Zweiphasenreaktionen gefunden, das in einem Rohrreaktor - gegebenenfalls gefüllt mit Füllkörpern oder Einbauten - durchgeführt werden kann und zur Aldolkondensation von Aldehyden zu ungesättigten Aldehyden unter hohen Raum-Zeit-Ausbeuten und Selektivitäten geeignet ist.

Im erfindungsgemäßen Verfahren kann die Aldolkondensation eines Aldehyds mit sich selbst durchgeführt werden, d. h. es wird nur ein Aldehyd eingesetzt. Es kann auch ein Gemisch mehrerer Aldehyde, die auch in unterschiedlichen Phasen der Mehrphasenreaktion vorliegen können, eingesetzt werden.

Wird ein Gemisch von mehreren Aldehyden eingesetzt, so können diese Aldehyde eine gleiche oder eine unterschiedliche Anzahl von Kohlenstoffatomen aufweisen.

In jedem Fall muß mindestens ein umzusetzender Aldehyd in der dispergierten Phase vorliegen. Mit dem erfindungsgemäßen Verfahren zur Aldolkondensation können Aldehyde mit 1 bis 15, bevorzugt 4 bis 15, besonders bevorzugt 4 bis 6 Kohlenstoffatomen zu den entsprechenden ungesättigten Aldehyden umgesetzt werden.

Der im erfindungsgemäßen Verfahren eingesetzte Rohrreaktor kann Füllkörper oder Einbauten enthalten. Füllkörper im Sinne der vorliegenden Erfindung sind beispielsweise: Raschigringe, Sättel, Pallringe, Telleretten, Maschendrahtringe oder Maschendrahtgewebe. Beispiele für Einbauten sind z. B. Filterplatten, Strombrecher, Kolonnenböden, Lochbleche oder sonstige Mischvorrichtungen. Als Einbauten im Sinne der vorliegenden Erfindung sind aber auch mehrere enge, parallel geschaltete Rohre unter Ausbildung eines Viellrohrreaktors einsetzbar. Besonders bevorzugt sind strukturierte Mischerpackungen oder Demisterpackungen.

Entscheidende Bedeutung hat im erfindungsgemäßen Verfahren die Einhaltung bzw. Überschreitung einer minimalen Querschnittsbelastung bzw. des Belastungsfaktors B des Rohrreaktors. Bei Aufwärtsbetrieb des Reaktors (Strömungsrichtung von unten nach oben) sollte der Flutpunkt überschritten werden. Der Reaktor wird also oberhalb des Punktes betrieben, bei dem man üblicherweise Blasensäulen betreibt. Bei Abwärtsbetrieb (Strömungsrichtung von oben nach unten) ist die Querschnittsbelastung so einzustellen, daß der Reaktor vollständig geflutet ist. Man arbeitet somit oberhalb des Punktes, bei dem man noch von einer Rieselphase (trickle bed) sprechen kann.

Zur genaueren Festlegung der minimal einzuhaltenden Belastung des Reaktors wird der Belastungsfaktor B des Rohrreaktors als ein dimensionsloser Druckverlust mit

$$B = PD/PS$$

berechnet, wobei PD [Pa/m] ein längenbezogener Druckverlust über den Reaktor unter Betriebsbedingungen und PS [Pa/m] eine Rechengröße mit der Dimension eines längenbezogenen Druckes, definiert als Verhältnis von Massenstrom M [kg/s] aller Komponenten im Reaktor zum Volumenstrom V [m$^3$/s] aller Komponenten unter Betriebsbedingungen, multipliziert mit g = 9,81 m/s$^2$, d. h. PS = (M/V)*g, bedeuten. Anschaulich wäre PS der statische Druck pro Meter eines Mehrphasengemisches in einem senkrecht stehenden Rohr, wenn alle Phasen mit gleicher Geschwindigkeit strömen würden. PS ist eine reine Rechengröße, die sich aus den dem Reaktor zugeführten Mengenströmen ergibt und die unabhängig von der Strömungsrichtung des Reaktors, der Strömungsgeschwindigkeit aller Phasen oder des Flutzustandes des Reaktors angegeben werden kann.

Der Druckverlust PD [Pa/m] wird als Rechengröße, um die Prozeßbedingungen festzulegen, verwendet und kann nach den gängigen Methoden für Ein- bzw. Mehrphasenströmungen berechnet werden. Gängige Verfahren zur Berechnung des Druckverlustes PD in Rohren, Einbauten oder Füllkörperschüttungen usw. können z. B. im VDI-Wärmeatlas 7. Erweiterte Auflage, VDI-Verlag GmbH, Düsseldorf 1994, Abschnitte Lal bis Lgb7, sowie im Standardwerk Heinz Brauer, Grundlagen der Einphasen- und Mehrphasenströmungen, Verlag Sauerländer, Aarau und Frankfurt am Main, 1971, nachgeschlagen werden.

Der Druckverlust PD ist bei der Einphasenströmung durch ein leeres Rohr durch

$$PD = Cw*\rho/2*w^2/D$$

mit

| | | |
|---|---|---|
| Cw | [-] | Widerstandsbeiwert des durchströmten Rohres |
| D | [m] | Rohrdurchmesser und |
| $\rho$ | [kg/m$^3$] | Dichte des strömenden Mediums unter Betriebsbedingungen, |
| w | [m/s] | Strömungsgeschwindigkeit Volumenstrom/Querschnittsfläche), |

gegeben.

Bei einer Strömung durch Füllkörper, Schüttungen oder Einbauten ist die Geschwindigkeit w durch die Effektivgeschwindigkeit (w/$\psi$) sowie der Rohrdurchmesser D durch den hydraulischen Kanaldurchmesser $d_H$ der Füllkörper oder Einbauten zu ersetzen, so daß gilt:

$$PD = Cw*\rho/2*(w/\psi)^2*1/d_H$$

mit

| | | |
|---|---|---|
| $d_H$ | [m] | Hydraulischer Kanaldurchmesser |
| $\psi$ | [-] | Leerrohranteil |
| Cw | [-] | Widerstandsbeiwert des durchströmten Apparates mit Füllung |

Die füllkörperspezifischen Daten $d_H$ und $\psi$ sind häufig Bestandteil der Lieferspezifikationen von Füllkörpern. Für eine Reihe von Füllkörpern sind Daten im oben erwähnten VDI-Wärmeatlas angegeben.

Der Leerrohranteil $\Psi$ kann auch experimentell bestimmt werden, indem man zum Beispiel den Reaktor vor und nach Befüllung mit den Füllkörpern auslitert. Der hydraulische Kanaldurchmesser wiederum kann, wenn er nicht bekannt ist, aus der spezifischen Oberfläche F [m$^2$/m$^3$] der Füllkörper oder Einbauten (in der Regel bekannt oder experimentell bestimmbar) nach der einfachen Beziehung

$$d_H = 4\psi/F$$

berechnet werden.

Der Widerstandsbeiwert von Rohren, Einbauten und Füllkörpern wird in der Regel in Abhängigkeit von der Reynoldszahl Re beschrieben, die Auskunft über den Strömungszustand unter den gewählten Bedingungen gibt. Bei Füllkörpern, Einbauten usw. ist fast immer folgende Beziehung anwendbar:

$$Cw = K_1/Re^n + K_2/Re^m$$

wobei häufig $n = 1$, $m = 0$ (Ansatz nach S. Ergun, Chem. Engng. Progr. 48, (1948), 89), oder $n = 1$, $m = 0,1$ (Ansatz nach Brauer et al.) verwendet wird. $K_1$, $K_2$ sind füllkörperspezifische Konstanten, die aus Lieferdaten oder aus der Literatur bekannt sind (Beispiele sind im VDI-Wärmeatlas und bei Brauer et al. zu finden). Sie können aber auch experimentell bestimmt werden, indem man den Rohrreaktor mit Füllkörpern mit einer Flüssigkeit unter verschiedenen Geschwindigkeiten betreibt und aus den bekannten Daten und dem gemessenen Druckverlust Cw in Abhängigkeit von Re bestimmt.

Die dimensionslose Reynoldszahl Re schließlich ist definiert als $Re = w*(\rho/\eta)*D$ für Leerrohre bzw. $Re = (w/\psi) * (\rho/\eta)*d_H$ für Rohre mit Einbauten oder Füllkörpern. $\eta$ [Pa*s] bezeichnet jeweils die Viskosität und $\rho$ [kg/m$^3$] die Dichte des strömenden Mediums.

Der Druckverlust bei Zweiphasenströmungen (hier flüssig-flüssig für Aldehyd/Katalysatorlösung) steigt überproportional an. Meist wird nach Lockhart-Martinelli (in Brauer et al.) der Druckverlust der Zweiphasenströmung $P_{III2}$ auf den Druckverlust einer der beiden Phasen bezogen, zum Beispiel auf den Druckverlust der reinen strömenden flüssigen Katalysatorphase $P_{I1}$, und in Beziehung zu dem Verhältnis des Druckverlustes der anderen als allein strömend gedachten Phase $P_{I2}$ gesetzt.

Zur Berechnung von Druckverlusten in Zweiphasenströmungen werden häufig dimensionslose Drücke nach $\phi^2 = P_{III2}/P_{I1}$ und $X^2 = P_{I1}/P_{I2}$ eingesetzt. Der weitere Zusammenhang $\varphi^2 = \text{Funktion}(X^2)$ ist vielfach untersucht. Beispiele finden sich in folgenden Literaturstellen:

Y. Sato, T.Hirose, F. Takahashi, M. Toda: "Pressure Loss and Liquid Hold Up in Packed Bed Reactor with Cocurrent Gas-Liquid Down Flow"; J. Chem. Eng. Of Japan, Vol 6 (Nr. 2), 1973, 147-152;
D. Sweeney: "A Correlation for Pressure Drop in Two-Phase Concurrent Flow in Packed Beds"; AIChE-Journal, Vol. 13, 7/1967, 663-669;
V. W. Weekman, J. E. Myers: "Fluid-Flow Characteristics of Concurrent Gas-Liquid Flow in Packed Beds"; AIChE-Journal, Vol 10 (Nr. 6), 11/1964, 951-957;
R. P. Larkins, R. P. White, D. W. Jeffrey: "Two-Phase Concurrent Flow in Packed Beds"; AIChE-Journal, Vol 7 (Nr. 2), 6/1961, 231-239 oder
N. Midoux, M. Favier, J.- C. Charpentier: " Flow Pattern, Pressure Loss and Liquid Holdup Data in Gas- Liquid Down-Flow Packed Beds with Foaming and Nonfoaming Liquids"; J. Chem. Eng. Of Japan, Vol 9 (Nr. 5), 1976, 350-356.

Häufig wird für die Berechnung der von Midoux vorgeschlagende Zusammenhang benutzt. Beispielsweise gilt

$$\Phi^2 = 1 + 1/X + 1,14 \, X^{0,54}$$

Dieser nach Lockart-Martinelli benannte Zusammenhang ist in vielen Werken graphisch dargestellt, detaillierte Abhandlungen hierüber finden sich in vielen Lehrbüchern der Verfahrenstechnik und Veröffentlichungen, so auch bei Brauer et al.

Der Druckverlust der Zweiphasenströmung $P_{III2}$ ergibt sich aus dem experimentell bestimmten oder wie oben erläutert abgeschätzten Druckverlust der reinen strömenden Flüssigphase $P_{I1}$ dann mit

$$P_{l1l2} = \phi^2 * P_{l1}$$

Allgemein gilt mit der Reaktorlänge L [m]

$$PD = P_{l1l2}/L$$

Der Druckverlust einer Mehrphasenströmung ist somit durch übliche Mittel der chemischen Verfahrenstechnik berechenbar. Analoges gilt für den zuvor definierten dimensionslosen Druckverlust B, d. h. den Belastungsfaktor des Mehrphasenreaktors.

Die Größe des dimensionslosen Belastungsfaktors B stellt eine notwendige Grundbedingung des erfindungsgemäßen Verfahrens dar; B sollte größer oder gleich 0,8, bevorzugt größer oder gleich 0,9 oder besonders bevorzugt größer oder gleich 1 sein.

Im Bereich B größer oder gleich 0,8 beginnt ein von oben nach unten betriebener Reaktor zu fluten. Es sei ausdrücklich darauf hingewiesen, daß bei Einhaltung dieser Bedingungen die Vorteile des erfindungsgemäßen Verfahrens auch dann erzielt werden, wenn der Reaktor von unten nach oben oder in anderer Richtung betrieben wird.

Höhere Querschnittsbelastungen des Reaktors (B >> 1), erkennbar am steigenden Differenzdruck über den Reaktor, sind jederzeit möglich und sogar erwünscht, solange die steigenden Raum-Zeit-Ausbeuten den gleichermaßen steigenden Energieverbrauch rechtfertigen. Eine Obergrenze ist daher nur durch praktische Überlegungen wie Energieverbrauch oder Schwierigkeiten bei der Trennung der Phasen nach erfolgter Reaktion gegeben.

Es ist somit ersichtlich, daß neben den Volumenströmen der einzelnen Phasen bzw. den hieraus abgeleiteten Leerrohrgeschwindigkeiten $w = V/(\pi D^2/4)$ die Apparateabmessungen des Reaktors (Länge L, Durchmesser D) sowie insbesondere die Daten der verwendeten Füllkörper (hydraulischer Durchmesser $d_H$, Leerrohranteil $\Psi$) eine wichtige Rolle spielen. Mit Hilfe dieser Parameter kann das Verfahren unschwer an die unterschiedlichsten Erfordernisse angepaßt werden, wichtig ist nur die Einhaltung der Forderung B >= 0,8, bevorzugt B >= 0,9 und besonders bevorzugt B >= 1.

Bei einer langsamen Reaktion wird man beispielsweise den hydraulischen Durchmesser der Füllkörper klein bzw. ihre spezifische Oberfläche groß wählen, so daß die geforderten Bedingungen für B schon bei kleinen Strömungsgeschwindigkeiten erreicht werden. Man erhält auf diese Weise ausreichende Verweilzeiten über die Länge eines technisch vernünftig dimensionierten Reaktors. Bei sehr schnellen Reaktionen empfiehlt sich eine umgekehrte Verfahrensweise.

Ein weiteres Kriterium bei der Durchführung des erfindungsgemäßen Verfahrens ist das Verhältnis des Massenstroms der flüssigen, den Katalysator enthaltenden Phase $M_1$ zu dem Massenstrom der dispersen Phasen $M_2$. Im erfindungsgemäßen Verfahren zur Durchführung von Aldolkondensationen ist der Massenstrom der Katalysatorphase $M_1$ größer als der Massenstrom $M_2$ der dispersen Phase oder Phasen. Im erfindungsgemäßen Verfahren kann das Massenverhältnis $M_1/M_2$ der kontinuierlichen Phase ($M_1$) zu der oder den dispersen Phasen ($M_2$) größer 2 sein, vorzugsweise gilt $M_1/M_2 > 10$. Strömungsverhältnisse mit $M_1/M_2 > 100$ sind durchaus möglich und häufig sogar vorteilhaft. Unter der Bedingung $M_1/M_2 > 2$ ist die Katalysatorphase die kontinuierliche Phase, während die disperse Phase oder die dispersen Phasen in feine Tropfen zerteilt wird.

Das erfindungsgemäße Verfahren wird vorteilhaft so ausgeführt, das mindestens eine Phase, die einen Aldehyd enthält, durch die von der kontinuierlichen Phase in den Rohrreaktor eingebracht Energie dispergiert wird.

Die Größe der so erhaltenen Tropfen können mit den üblichen ingenieurtechnischen Mitteln abgeschätzt werden. Es eignen sich hierfür Ansätze mit dimensionslosen Kennzahlen wie

$$d_S/d_H = k * Re_{l1l2}{}^m * We_{l1l2}{}^n$$

mit

| | |
|---|---|
| $d_S$ | Durchmesser der Tropfen nach Sauter (in Brauer et al. beschrieben), |
| $d_H$ | hydraulischer Füllkörperdurchmesser, |
| $Re_{l1l2}$ | Reynoldszahl der Mehrphasenströmung $= w_{l1l2} * (\rho_{ll}/\eta_{ll}) * (d_H/\Psi)$, |
| Welle | Weberzahl der Mehrphasenströmung $= w_{l1l2}{}^2 * (\rho_{ll}/\sigma_{l1l2}) * (d_H/\Psi)^2$, |
| k, m, n | empirische Konstanten (bekannt oder durch Versuche zu ermitteln), |
| w | Leerrohrgeschwindigkeiten $[m/s] = V/(\pi D^2/4)$, |

V          Volumenstrom unter Betriebsbedingungen [$m^3/s$],

$\rho$          Dichte unter Betriebsbedingungen [$kg/m^3$],

$\eta$          Viskosität unter Betriebsbedingungen [Pa*s] und

$\gamma$          Grenzflächenspannung unter Betriebsbedingungen [N/m]

und den Indices l1 (erste Flüssigphase) und l2 (zweite Flüssigphase)

Bei strukturierten Packungen wie Sulzer-SMV oder engen Rohren als Einbauten scheint es plausibel, daß ein berechneter Tropfendurchmesser ds größer als der Kanaldurchmesser nicht sinnvoll ist. Dies gilt aber nicht für durchlässige Packungen und Füllkörper wie beispielsweise Maschendrahtringe oder Maschendrahtgewebe (sogenannte Demisterpackungen oder Tropfenabscheider). Im erfindungsgemäßen Verfahren können berechnete Tropfendurchmesser verwendet werden, die mindestens gleich oder kleiner als der hydraulische Kanaldurchmesser sind:

$$d_S/d_H \leq 1, \text{ vorzugsweise} < 0{,}9.$$

Aus dem berechneten Tropfendurchmesser läßt sich schließlich eine Stoffübergangsfläche nach

$$A_s = 6\varphi_{l2}d_s \; [m^2/m^3]$$

berechnen.

Für den Phasenanteil $\phi_{l2}$ der dispersen Phase (im Falle der Aldolkondensation ist eine, zumindest einen Aldehyd enthaltende organische Phase dispergiert), kann mit den Leerrohrgeschwindigkeiten der Phasen

$$\varphi_{l2} \sim w_{l2}/w_{lIl2}$$

gesetzt werden.

Die Verweilzeit T der den Reaktor durchströmenden Phasen läßt sich angenähert nach $\tau \sim L*\psi/w_{lIl2}$. berechnen. Die Verweilzeit T beträgt bei dem erfindungsgemäßen Verfahren in der Regel weit unter einer Stunde und kann im Minutenbereich oder sogar noch darunter liegen. Dennoch werden bei dieser völlig ungewöhnlichen Fahrweise - hoher Katalysatordurchsatz im Reaktor, vergleichsweise geringer Anteil an Edukt an der Reaktionsmasse, dadurch bedingt wiederum sehr kurze Verweilzeit überraschend hohe Raum-Zeit-Ausbeuten erzielt. Alternativ kann bei gleichen Raum-Zeit-Ausbeuten bei deutlich tieferen Temperaturen als üblich gearbeitet werden, da die Steigerung der Reaktionsgeschwindigkeit, was zum Beispiel die Minimierung von Folgereaktionen und damit verbesserte Selektivität nach sich ziehen kann, dies wirtschaftlich zuläßt.

Das erfindungsgemäße Verfahren kann sehr flexibel den unterschiedlichsten Anforderungen angepaßt werden. Für spezielle Anforderungen bieten sich folgende Ausführungsformen des erfindungsgemäßen Verfahrens an:

Erfordert der Einsatzzweck eine sehr lange Durchmischungszone oder sind Ruhezonen z. B. zur Abnahme von Stoffströmen erforderlich, so bietet sich eine kaskadierende Anordnung von Rohrreaktoren mit Einbauten oder Füllkörpern an.

Eine Kaskadierung von Rohrreaktoren oder die alternative Anordnung von gepackten und leeren Rohrabschnitten ist zu empfehlen, wenn ein besonders geringer Druckverlust gewünscht wird.

Weiterhin ist die parallele Anordnung von Rohrreaktoren oder die Verwendung eines Vielrohrreaktors, wobei die Rohre die Funktion der Einbauten übernehmen können, einsetzbar.

Auch die Wärmeabfuhr bei stark exothermen Reaktionen wie zum Beispiel bei der Aldolkondensation ist beim erfindungsgemäßen Verfahren unkritisch. Der hohe Durchsatz des Katalysatorkreislaufs wirkt als Wärmeträger, so daß selbst bei adiabatischer Fahrweise des Reaktors nur geringe Temperaturdifferenzen auftreten und eine homogene Temperaturverteilung im Reaktor ohne Temperaturspitzen resultiert. Die erzeugte Wärme läßt sich dann bequem durch einen beliebig im äußeren Katalysatorkreislauf angeordneten, konventionellen Wärmetauscher abführen oder zur Energiegewinnung ausnutzen. Zur besseren Wärmeabfuhr kann es unter Umständen günstig sein, den Katalysatorkreislauf noch höher zu fahren (also bei einem höheren B-Wert) als technisch notwendig ist, da über den Katalysatorkreislauf ein kleiner Temperaturgradient über den Reaktor einstellbar ist.

Das erfindungsgemäße Verfahren bietet im Vergleich zum Stand der Technik erhebliche Vorteile, genannt seien:

- Bei vergleichsweise niedrigen Temperaturen können hohe Raum-Zeit-Ausbeuten erreicht werden.
- Die Bildung von Nebenprodukten ist extrem niedrig.
- Der Katalysator wird geschont, die Desaktivierung ist sehr gering, eine kontinuierliche Ausschleusung wird minimiert.

Bei der erfindungsgemäßen Herstellung von $\alpha$?$\beta$-ungesättigten Aldehyden durch Aldolkondensation von Aldehyden kommt als weiterer Vorteil hinzu, daß aufgrund der hohen Reaktionsgeschwindigkeiten auch Aldehyde mit sehr geringer Löslichkeit in der Katalysatorphase wirtschaftlich zu den entsprechenden Aldolkondensationsprodukten umgesetzt werden können.

Als Lösungsmittel für die Herstellung der Katalysatorlösung bzw. -phase sind alle diejenigen Solventien geeignet, die folgende Bedingungen erfüllen:

- Das Lösungsmittel ist in der Produktphase wenig löslich.
- Das Produkt löst sich nur wenig in der Katalysatorphase, die aus Katalysator und Lösungsmittel besteht.
- Das Lösungsmittel hat für den eingesetzten Katalysator eine genügend hohe Löslichkeit.

Die kontinuierliche Phase des erfindungsgemäßen Verfahrens (d. h. die Katalysatorphase) kann weitgehend aus Wasser bestehen.

Optional kann die Katalysatorphase Phasentransfer-, oberflächenaktive- oder amphiphile Reagenzien oder Tenside enthalten.

In besonderen Fällen kann die kontinuierliche Phase vollständig aus Wasser bestehen. Weiterhin kann die kontinuierliche Phase weitgehend aus Wasser und einem wasserlöslichen Lösungsmittel bestehen. Als organisches Lösungsmittel können z. B. Propandiol, Glycerin, Diethylenglycol (DEG) oder Dimethylformamid eingesetzt werden.

Der Anteil an Wasser und organischem Lösungsmittel liegt bevorzugt über 60, besonders bevorzugt über 80 Gew.-% der Phase.

Als Katalysator im erfindungsgemäßen Verfahren können wasserlösliche, basische Verbindungen wie z. B. Hydroxide, Hydogencarbonate, Carbonate, Carboxylate oder ihre Gemische in Form ihrer Alkali- oder Erdalkaliverbindungen verwendet werden. Vorzugsweise kommen Alkalihydroxide wie Natriumhydroxid zum Einsatz.

Die Konzentration des Katalysators in der kontinuierlichen Phase liegt zwischen 0,1 und 15 Gew.-%, insbesondere zwischen 0,1 und 5 Gew.-%.

Das erfindungsgemäße Verfahren ist für die Umsetzung von Aldehyden oder Aldehydgemischen geeignet, die Kondensationsreaktionen eingehen können. Wird nur ein Aldehyd eingesetzt, so muß dieser zwei $\alpha$-Wasserstoffatome am gleichem Kohlenstoffatom in Nachbarschaft zur CO-Gruppe besitzen. Werden zwei oder mehrere unterschiedliche Aldehyde eingesetzt, so muß mindestens einer der Aldehyde zwei a-Wasserstoffatome am gleichen Kohlenstoffatom haben.

Aldehyde mit zwei $\alpha$-Wasserstoffatomen gemäß obiger Definition sind beispielsweise: Acetaldehyd, Propanal, n-Butyraldehyd, n-Valeraldehyd, 3-Methylbutyraldehyd, n-Hexanal, 3-Methylpentanal, 4-Methylpentanal, n-Heptanal, n-Octanal, n-Nonanal, n-Decanal. Diese eignen sich auch für eine Homokondensation.

Beispiele für Aldehyde mit einem $\alpha$-Wasserstoffatom nach obiger Definition sind: Isobutyraldehyd, 2-Methylbutyraldehyd, 2-Methylpentanal, 2-Ethylhexanal, Cyclohexylaldehyd. Beispiele für Aldehyde mit keinem $\alpha$-Wasserstoffatom sind: Benzaldehyd, 2,2-Dimethylpropanal und Formaldehyd. Die Aldehyde der beiden zuletzt genannten Gruppen können nur mit einem Aldehyd mit zwei $\alpha$-Wasserstoffatomen eine Aldolkondensation eingehen.

Bevorzugte Einsatzstoffe für das erfindungsgemäße Verfahren sind: n-Butyraldehyd, n-Valeraldehyd, ein Gemisch aus n-Butyraldehyd und Isobutyraldehyd, Gemische aus n-Valeraldehyd mit 2-Methylbutyraldehyd oder 3-Methylbutyraldehyd oder das entsprechende Dreikomponentengemisch. Ebenfalls kann ein Gemisch aus $C_4$- und $C_5$-Aldehyden oder ein Gemisch der isomeren ($C_9$-Aldehyde) Nonanale eingesetzt werden. Diese Aldehyde können z.B. durch Hydroformylierung von Olefinen hergestellt werden.

Bei Einsatz von mehr als einem Aldehyd oder einem Aldehydgemisch können die Einzelkomponenten getrennt in den Strom der Katalysatorlösung eingespeist werden. Ebenfalls ist es möglich, alle Edukte vor der Einspeisung zu mischen und gemeinsam einzuspeisen. Weiterhin können die Aldehyde als Lösung eingesetzt werden. Als Lösungsmittel können inerte, in der Katalysatorlösung kaum lösliche Flüssigkeiten wie z. B. Kohlenwasserstoffe (Pentan, Cyclohexan, Toluol) verwendet werden.

Weitere Gegenstände verbunden mit der vorliegenden Erfindung sind Verwendungen der durch das erfindungsgemäße Verfahren hergestellten Aldolkondensationsprodukte. Diese können durch Hydrierung zur Herstellung von gesättigten Alkoholen verwendet werden. Die so erhaltenen gesättigten Alkohole dienen wiederum zur Herstellung von Weichmachern, Detergentien oder Lösungsmitteln. Als Vorstufe für Weichmacheralkohole seien insbesondere die

ungesättigten C$_8$- und C$_{10}$-Aldehyde genannt.

Weiterhin können die Aldolkondensationsprodukte durch Selektivhydrierung in die gesättigten Aldehyde und diese durch eine anschließende Oxidation in Carbonsäuren überführt, d. h. zur Herstellung von Carbonsäuren verwendet werden.

Darüber hinaus werden die ungesättigten Aldehyden wegen ihrer Reaktivität bei vielen Synthesen eingesetzt. Ein weiteres Einsatzgebiet der gesättigten und ungesättigten Aldehyde ist die Verwendung als Riechstoff.

Das erfindungsgemäße Verfahren zur Aldolkondensation von Aldehyden kann in einem Temperaturbereich von 30 °C bis 200 °C, vorzugsweise im Bereich 60 °C bis 150 °C durchgeführt werden.

Das Reaktionsrohr kann im Gleichstrom von oben nach unten oder umgekehrt durchströmt werden. Aus Sicherheitsgründen wird der Beschickung von oben der Vorzug gegeben.

Die Reaktionswärme kann über verschiedene Wärmeaustauscher abgeführt werden. Die Wärmeaustauscher müssen hierbei nicht in der Nähe des Reaktionsraums sein, sondern können auch beliebig außerhalb des Reaktors liegen. Die einzelnen Wärmeflüsse sind abhängig von der spezifischen Reaktionswärme sowie von den gewünschten Temperaturen im Reaktor und in den Aufarbeitungsvorrichtungen.

Die abgeführte Reaktionswärme kann so sehr einfach genutzt werden, z.B. im Prozeß selbst, zur Beheizung einer Destillationseinrichtung oder zur Erzeugung von Dampf

Das den Reaktor verlassende Flüssigkeitsgemisch wird in einem Flüssig-Flüssig-Trennbehälter in Katalysatorphase und Produktphase mechanisch getrennt. Dies kann in Absitzbehältern verschiedener Bauart oder Zentrifugen erfolgen. Aus Kostengründen werden Absitzbehälter bevorzugt.

Die Verweilzeiten in der Trennvorrichtung sind zwar nicht grundsätzlich kritisch, werden aber vorteilhaft klein gehalten. Dies hat folgende Vorteile: Die Trennvorrichtung ist klein und das Investment dafür entsprechend gering. Bei kurzen Verweilzeiten treten praktisch keine Nebenreaktionen im Trennbehälter auf Damit die Trennung der Phasen schnell erfolgt, muß der Dichteunterschied der beiden Phasen genügend groß und deren Viskositäten gering sein. Alle drei Größen sind eine Funktion der Temperatur und können durch orientierende Versuche leicht ermittelt werden.

Darüber hinaus kann die Dichte und Viskosität der Katalysatorlösung durch Auswahl des Solvens und der Katalysatorkonzentration variiert werden. Als weitere Möglichkeit kann die Dichte und Viskosität der Produktphase durch Zusatz eines Lösungsmittel verändert werden. Die Phasentrennung kann in einem weiten Temperaturbereich vorgenommen werden. Dabei kann die Trenntemperatur auch höher sein als die Temperatur des Reaktionsaustrags am Reaktorausgang. Aus energetischen Gründen ist es jedoch nicht günstig, eine höhere Temperatur als die Flüssigkeitstemperatur am Reaktorausgang anzuwenden. Als tiefstmögliche Temperatur ist der Stockpunkt einer der beiden flüssigen Phasen anzusehen. Im Hinblick auf kurze Trennzeiten werden jedoch, wie oben erwähnt, keine zu tiefen Temperaturen gewählt.

Das entstehende Reaktionswasser verdünnt die Katalysatorlösung und muß daher ständig aus dem Prozeß entfernt werden. Dies kann durch Destillation des Reaktoraustrags erfolgen, wobei ein wasserhaltiges Azeotrop anfällt. Dieses kann wiederum in eine wäßrige und organische Phase getrennt werden. Ein Teil der wäßrigen Phase kann zum Ausgleich der Wasserbilanz ausgeschleust werden. Überschüssiges Wasser kann auch durch Destillation aus der abgetrennten Katalysatorphase entfernt werden. Darüber hinaus kann Reaktionswasser durch Entnahme eines Teils der Katalysatorlösung abgetrennt werden

Der Produktstrom kann nach Abtrennung durch bekannte Verfahren gereinigt werden, z.B. durch Destillation.

Die abgetrennte Katalysatorlösung wird, ggf. nach Ausschleusung einer kleinen Teilmenge und entsprechendem Ersatz durch frische Katalysatorlösung, in den Reaktor zurückgeführt.

Die folgenden Beispiele sollen die Erfindung beschreiben, ohne deren Anwendungsbreite einzuschränken, die aus den Patentansprüchen hervorgeht.

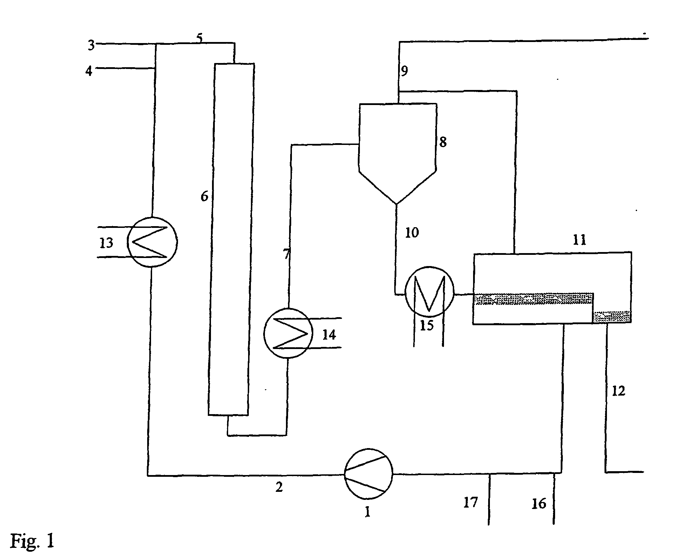

Die Aldolkondensation von Aldehyden gemäß dem Verfahren der Erfindung erfolgte in einer Versuchsapparatur, die schematisch in Fig. 1 dargestellt ist. Hierin wird mit einer Pumpe 1 die kontinuierliche Katalysatorphase 2 im Kreislauf gepumpt. Zum Katalysator wird der Aldehyd oder die Aldehydmischung durch Leitung 3 oder im Falle der getrennten Einspeisung von verschiedenen Aldehyden durch die Leitungen 3 und 4 zugemischt. In den nachfolgend aufgeführten Beispielen wurden die Edukte ausschließlich über Leitung 3 zugemischt. Die so erhaltene Mehrphasenmischung wurde bei den Beispielen 3 bis 14 durch Leitung 5 durch einen Rohrreaktor 6 mit einer Länge von 3 m und einem Durchmesser von 17,3 mm gepumpt, der mit statischen Mischelementen mit einem hydraulischen Durchmesser von 2 mm versehen war. In den Beispielen 1 und 2 wurden andere Reaktoren verwendet, deren Dimensionen dort genannt sind. Die resultierende Mischung, bestehend aus dem Reaktionsprodukt, nicht umgesetztem Edukt und dem Katalysator wurden durch Leitung 7 im optionalen Gasabscheider 8 von leicht flüchtigen Bestandteilen durch Ausschleusung über Leitung 9 befreit. Für die nachfolgend aufgeführten Beispiele, außer Beispiel 2, war diese Leitung geschlossen.

Der nach der Entgasung 8 anfallende Flüssigkeitsstrom 10 wurde in einen Phasentrennbehälter 11 geleitet. Hier wurde die wäßrige Katalysatorphase über Leitung 2 abgetrennt und erneut dem Kreislauf zugeführt. Die über ein Wehr gelaufene organische Phase, die das Reaktionsprodukt enthält, kann über Leitung 12 entnommen werden.

Die Reaktionswärme wurde über die außerhalb des Reaktors liegenden Wärmetauscher 13, 14 und 15 abgeführt.

Als Lösungsmittel für den Katalysator wurde Wasser oder ein Gemisch aus Wasser und Diethylenglykol (DEG) eingesetzt. Die Versuchspaare 3 und 4, 5 und 6, 7 und 8, 9 und 10, 11 und 12 sowie 13 und 14 dokumentieren deutlich die Umsatzsteigerung bei Verwendung eines Lösungsmittelgemisches.

Die den Beispielen 3 bis 14 beigefügten Tabellen beschreiben die Katalysatorzusammensetzung in Massenprozenten, die zugefahrene Menge Aldehyd und dessen Zusammensetzung in Flächenprozenten der gaschromatographischen Analyse.

Im unteren Bereich der jeweils zweiten Tabelle jedes Beispiels ist die Produktzusammensetzung ebenfalls in Flächenpozenten der gaschromatographischen Analyse aufgelistet. Für die Dokumentation der Beispiele 3-14 wurde aus Gründen der Übersicht nicht zwischen den Isomeren der einzelnen $C_5$-Säuren bzw. $C_5$-Alkoholen unterschieden. Diese Werte sind als "Alkohol" bzw. "Säure" zusammengefaßt. Ebenfalls zusammengefaßt als "Aldoladditionsverbindungen" sind die gesättigten 3-Hydroxyalkanale der $C_5$-Aldehyde. Die "Trimere" beschreiben den Anteil an Schwersiedern, die aus Aldolreaktion (Addition und Kondensation) von drei $C_5$-Aldehyden hervorgegangen sind.

Im oberen Bereich der jeweils zweiten Tabelle in den Beispielen sind die Raum-Zeit-Ausbeute (RZA), der Umsatz (U) der Aldehyde, die Selektivität (S) zu den gewünschten Aldolkondensationsprodukten und der Belastungsfaktor (B) des Rohrreaktors angegeben. Bei der beschriebenen Katalysatorzusammensetzung ist zu beachten, daß es sich bei den Beispielen 3 bis 14 um Startwerte handelt. Der Anteil an NaOH und optional DEG wurde durch das Reaktionswasser der Aldolkondensation leicht verdünnt. Darüber hinaus führt die parallel zu Aldolkondensation ablaufende Cannizzaro-Reaktion zur Neutralisation des alkalischen Katalysators. Beide Effekte sind im beobachteten Zeitraum aber so gering, daß dies für die Beschreibung der Versuche und der Versuchsergebnisse unwesentlich ist.

**Beispiel 1 (Vergleichsbeispiel):**

Herstellung von Propylheptenal aus n-Pentanal

Propylheptenal wurde durch Kondensation von n-Pentanal in einem Strömungsrohr hergestellt. Die Reaktorauslegung, das Phasenverhältnis zwischen organischer Phase und wäßriger Katalysatorphase und die Verweilzeit wurden in Anlehnung an ein großtechnisches Verfahren zur Herstellung von 2-Ethylhex-2-enal gewählt. Der Reaktor der oben beschriebenen Versuchsanlage (Fig. 1) wurde gegen ein 60 m-langes DN20-Rohr ausgetauscht. Das Reaktorvolumen betrug 19 l. (In einem hier nicht beschriebenen Vorversuch ist sichergestellt worden, daß mit diesem Reaktor ein großtechnisches Verfahren zur Herstellung von 2-Ethylhex-2-enal gut simuliert werden kann).

Der Katalysatorkreislauf betrug 500 l/h (2,7 %-ige wäßrige NaOH). n-Pentanal wurde mit einer Volumengeschwindigkeit von 50 l/h eingespeist.

Im kontinuierlichen Betrieb wurden folgende Ergebnisse erhalten:

|  | Beispiel 1a | Beispiel 1b |
|---|---|---|
| Temperatur | 95 °C | 130 °C |
| Druck | 1,0bar | 2,7 bar |
| n-Pentanal | 24,0 Mol % | 3,0 Mol-% |
| 2-Propylheptenal | 69, 5 Mol-% | 94,4 Mol-% |
| Rest (hauptsächlich Hochsieder) | 6,5 Mol% | 2,6 Mol-% |
| Raum-Zeit-Ausbeute | 1,3 t/(m³*h) | 1,8 t/(m³*h) |

Der Belastungsfaktor B betrug 0,02 .

**Beispiel 2**

Herstellung von 2-Propylheptenal

Als Reaktor wurde ein 995 mm langes DN15-Rohr (Innendurchmesser 17,9 mm) verwendet. Der Reaktor war mit Maschendrahtringen (Vereinigte Füllkörperfabriken, VFF, 4*4 mm mit Steg, Oberfläche ca. 2100 m²/m³) gefüllt, wie sie zur Destillation eingesetzt werden. Experimentell wurde in dem gefüllten Reaktor ein Anteil der Füllkörper am Reaktorvolumen von 8,5 % bestimmt. Der Leerrohranteil war somit 91,5 %, das entsprach einem freien Reaktorvolumen von 229 cm³. Der wirksame hydraulische Durchmesser wurde mit $d_H$ =1,913 mm berechnet.

Während des kontinuierlichen Versuchs wurde der NaOH-Gehalt in der Katalysatorlösung durch Zugabe von 1-molarer Natronlauge auf 2,85 Gew.-% gehalten.

Der Katalysatorkreislauf betrug 400 l/h, n-Pentanal wurde mit einer Volumengeschwindigkeit von 1,0 l/h eingespeist. Zur Inertisierung wurde zusätzlich eine geringe Menge Stickstoff (0,5 Mol/h) zudosiert.

Der im Rohprodukt enthaltene Stickstoff entweicht zusammen mit einem Azeotrop aus Wasser, n-Pentanal und den Produkten aus dem Gasabscheider 8 durch Leitung 9. Dieser Strom wird gekühlt (Kühler und Abscheider sind in Figur 1 nicht dargestellt). Dabei fällt eine organische Phase und eine wäßrige Phase an. Die organische Phase wird in den Produktabscheider 11 geleitet. Die wäßrige Phase wurde verworfen.

Der Versuch wurde bei 1 bar und 95°C durchgeführt. Der Belastungsfaktor B betrug 9,92.

Im Gleichgewicht wurde ein Produkt mit folgender durchschnittlicher Zusammensetzung erhalten:

| | Edukt | Produkt |
|---|---|---|
| n-Pentanal | 97,7 Mol-% | 7,7 Mol-% |
| Propylheptenal | | 89,8 Mol-% |
| Rest (hauptsächlich Hochsieder) | 2,3% Mol-% | 2,5 Mol-% |

Die Raum-Zeit-Ausbeute berechnete sich zu 2,6 t/(m³*h).

Dieser Versuch zeigte, daß bereits bei 95°C in einem nur 1 m langen Rohr ein hoher Umsatz bei geringer Bildung von Hochsiedern erreicht wird. Das erfindungsgemäße Verfahren lieferte höhere Raum-Zeit-Ausbeuten als ein konventionelles Verfahren (Vergleichsbeispiel 1).

**Beispiel 3:**

Dieses Beispiel beschreibt das erfindungsgemäße Verfahren für die Aldolkondensation von n-Pentanal zu 2-Propylheptenal (I+I-Produkt). Der Reaktor wurde mit einer Katalysatorbelastung von 400 kg/h bei einer Temperatur von 110°C bei Eigendruck der Reaktionsteilnehmer durchströmt. Als Reaktor wurde ein 3 m langes DN15-Rohr (Innendurchmesser 17,3 mm) verwendet. Der Reaktor war mit Füllkörpern der Firma Sulzer mit einem hydraulischen Durchmesser von 2 mm (SMV 2) gefüllt.

| Katalysator | |
|---|---|
| c NaOH | 2,34 Gew.-% |
| Wasser | 97,66 Gew.-% |
| Eduktstrom [l/h] | 4,11 |
| Eduktzusammensetzung | |
| n-Pentanal | 96,1 Gew.-% |
| Aldole | 0,9 Gew.-% |
| Trimere | 1,4 Gew.-% |
| Rest | 1,6 Gew.-% |

Folgendes Ergebnis wurde erzielt:

| | |
|---|---|
| RZA [t/m³/h] | 3,9 |
| Umsatz | 97,6% |
| Selektivität | 96,2% |
| B | 15,34 |
| n-Pentanal (nicht umgesetztes n-Pentanal) | 2,3 Gew.-% |
| 2-Propylheptenal | 94,0 Gew.-% |
| Aldoladditionsprodukte | 0,7 Gew.-% |

(fortgesetzt)

| Trimere | 1,0 Gew.-% |
|---|---|
| Rest | 2,0 Gew.-% |

**Beispiel 4:**

Dieses Beispiel beschreibt das erfindungsgemäße Verfahren für die Aldolkondensation von n-Pentanal zu 2-Propylhepenal (1+1-Produkt) unter Einsatz des Cosolvens Diethylenglykol (DEG). Der Reaktor (gemäß Beispiel 3) wurde mit einer Katalysatorbelastung von 400 kg/h bei einer Temperatur von 110°C bei Eigendruck der Reaktionsteilnehmer durchströmt.

| Katalysator | |
|---|---|
| c NaOH | 3,76 Gew.-% |
| Diethylenglykol | 50,00 Gew.-% |
| Wasser | 46,24 Gew.-% |
| Eduktstrom [l/h] | 4,02 |
| Eduktzusammensetzung | |
| n-Pentanal | 96,1 Gew.-% |
| Aldole | 1,3 Gew.-% |
| Trimere | 0,8 Gew.-% |
| Rest | 1,7 Gew.-% |

Folgendes Ergebnis wurde erzielt:

| RZA [t/m$^3$/h] | 3,9 |
|---|---|
| Umsatz | 99,5 % |
| Selektivität | 95,9 % |
| B | 13,91 |
| n-Pentanal (nicht umgesetztes n-Pentanal) | 0,5 Gew.-% |
| 2-Propylheptenal | 95,4 Gew.-% |
| Aldoladditionsprodulcte | 0,1 Gew.-% |
| Trimere | 3,0 Gew.-% |
| Rest | 0,9 Gew.-% |

**Beispiel 5:**

Dieses Beispiel beschreibt das erfindungsgemäße Verfahren für die Aldolkondensation von 3-Methylbutanal (3-MBA) zu 2-Isopropyl-5-methylhexenal (3+3). Der Reaktor (gemäß Beispiel 3) wurde mit einer Katalysatorbelastung von 400 kg/h bei einer Temperatur von 110°C bei Eigendruck der Reaktionsteilnehmer durchströmt.

| Katalysator | |
|---|---|
| c NaOH | 2,44 Gew.-% |
| Wasser | 97,56 Gew.-% |
| Eduktstrom [l/h] | 4,27 |

(fortgesetzt)

| Eduktzusammensetzung | |
|---|---|
| 3-Methylbutanal | 98,7 Gew.-% |
| Aldole | 0,2 Gew.-% |
| Trimere | 0,1 Gew.-% |
| Rest | 0,1 Gew.-% |

Folgendes Ergebnis wurde erzielt:

| RZA [t/m$^3$/h] | 3,2 |
|---|---|
| Umsatz | 78,2 % |
| Selektivität | 93,3 % |
| B | 15,48 |
| 3-Methylbutanal | 21,6 Gew.-% |
| Alkohol | 1,2 Gew.-% |
| 2-Isopropyl-5-methylhexenal | 72,0 Gew.-% |
| Aldoladditionsprodukte | 1,1 Gew.-% |
| Trimere | 1,5 Gew.-% |
| Rest | 2,6 Gew.-% |

**Beispiel 6:**

Dieses Beispiel beschreibt das erfindungsgemäße Verfahren für die Aldolkondensation von 3-Methylbutanal (3-MBA) zu 2-Isopropyl-5-methylhexenal (3+3) unter Einsatz des Cosolvens Diethylenglykol (DEG). Der Reaktor (gemäß Beispiel 3) wurde mit einer Katalysatorbelastung von 400 kg/h bei einer Temperatur von 110 °C bei Eigendruck der Reaktionsteilnehmer durchströmt.

| Katalysator | |
|---|---|
| c NaOH | 3,46 Gew.-% |
| Diethylenglykol | 50,00 Gew.-% |
| Wasser | 46,54 % |
| Eduktstrom [l/h] | 4,00 |
| Eduktzusammensetzung | |
| 3-Methylbutanal | 98,9 Gew.-% |
| Aldoladditionsprodukte | 0,2 Gew.-% |
| Trimere | 0,1 Gew.-% |
| Rest | 0,8 Gew.-% |

Folgendes Ergebnis wurde erzielt:

| RZA [t/m$^3$/h] | 3,7 |
|---|---|
| Umsatz | 95, 7 % |
| Selektivität | 95,2 % |

(fortgesetzt)

| B | 13,91 |
|---|---|
| 3-Methylbutanal | 4,2 Gew.-% |
| Alkohol | 0,5 Gew.-% |
| 2-Isopropyl-5-methylhexenal | 90,1 Gew.-% |
| Trimere | 2,6 Gew.-% |
| Rest | 2,6 Gew.-% |

**Beispiel 7:**

Dieses Beispiel beschreibt das erfindungsgemäße Verfahren für die Coaldolkondensation von n-Pentanal und 2-Methylbutanal (2-MBA) zu einem Isodecenal, bestehend aus den Isomeren 2-Propyl-4-methylhexenal (1+2) sowie 2-Propylheptenal (1+1). Der Reaktor (gemäß Beispiel 3) wurde mit einer Katalysatorbelastung von 400 kg/h bei einer Temperatur von 110°C bei Eigendruck der Reaktionsteilnehmer durchströmt.

| Katalysator | |
|---|---|
| c NaOH | 2,27 Gew:-% |
| Wasser | 97,73 % |
| Edukstrom [l/h] | 4,17 |
| Eduktzusammensetzung | |
| 2-Methylbutanal | 51,3 Gew.-% |
| n-Pentanal | 46,0 Gew.-% |
| Säure | 0,3 Gew.-% |
| Aldole | 0,9 Gew.-% |
| Trimere | 0,1 Gew.-% |
| Rest | 1,3 Gew.-% |

Folgendes Ergebnis wurde erzielt:

| RZA [t/m$^3$/h] | 2,3 |
|---|---|
| Umsatz | 57,7 % |
| Selektivität | 94,8 % |
| B | 15,40 |
| 2-Methylbutanal | 33,0 Gew.-% |
| n-Pentanal | 3,1 Gew.-% |
| Alkohol | 0,3 Gew.-% |
| Säure | 1,2 Gew.-% |
| 2-Propyl-4-methylhexenal | 24,4 Gew.-% |
| 2-Propylheptenal | 34,3 Gew.-% |
| Aldoladditionsprodukte | 1,4 |
| Trimere | 1,1 |
| Rest | 1,4 |

**Beispiel 8:**

Dieses Beispiel beschreibt das erfindungsgemäße Verfahren für die Coaldolkondensation von n-Pentanal und 2-Methylbutanal (2-MBA) zu einem Isodecenal, bestehend aus den Isomeren 2-Propyl-4-methylhexenal (1+2) sowie 2-Propylheptenal (1+1), unter Einsatz des Cosolvens Diethylenglykol (DEG). Der Reaktor (gemäß Beispiel 3) wurde mit einer Katalysatorbelastung von 400 kg/h bei einer Temperatur von 110°C bei Eigendruck der Reaktionsteilnehmer durchströmt.

| Katalysator | |
|---|---|
| c NaOH | 3,25 Gew.-% |
| Diethylenglykol | 45,00 Gew.-% |
| Wasser | 51,75 Gew.-% |
| Eduktstrom [1/h] | 3,66 |
| Eduktzusammensetzung | |
| 2-Methylbutanal | 45,4 Gew.-% |
| n-Pentanal | 51,2 Gew.-% |
| Säure | 0,4 Gew.-% |
| Aldole | 1,0 Gew.-% |
| Trimere | 0,1 Gew.-% |
| Rest | 1,8 Gew.-% |

Folgendes Ergebnis wurde erzielt:

| RZA [t/m$^3$/h] | 2,7 |
|---|---|
| Umsatz | 77,0 % |
| Selektivität | 94,9 % |
| B | 13, 75 |
| 2-Methylbutanal | 18,4 Gew.-% |
| n-Pentanal | 0,3 Gew.-% |
| Alkohol | 0,8 Gew.-% |
| Säure | 0,9 Gew.-% |
| 2-Propyl-4-methylhexenal | 45,2 Gew.-% |
| 2-Propylheptenal | 28,5 Gew.-% |
| Aldoladditionsprodukte | 0,9 Gew.-% |
| Trimere | 4,1 Gew.-% |
| Rest | 1,2 Gew.-% |

**Beispiel 9:**

Dieses Beispiel beschreibt das erfindungsgemäße Verfahren für die Coaldolkondensation von n-Pentanal und 3-Methylbutanal (3-MBA) zu einem Isodecenal, bestehend aus den Isomeren 2-Propyl-5-methylhexenal und 2-Isopropylheptenal (1+3) sowie 2-Propylheptenal (1+1). Der Reaktor (gemäß Beispiel 3) wurde mit einer Katalysatorbelastung von 400 kg/h bei einer Temperatur von 110°C bei Eigendruck der Reaktionsteilnehmer durchströmt.

| Katalysator | |
|---|---|
| c NaOH | 2,13 Gew.-% |
| Wasser | 97;87 Gew.-% |
| Edukstrom [l/h] | 4,14 |
| Eduktzusammensetzung | |
| 3-Methylbutanal | 45,8 Gew.-% |
| n-Pentanal | 52,4 Gew.-% |
| Säure | 0,3 Gew.-% |
| Aldole | 0,9 Gew.-% |
| Trimere | 0,1 Gew.-% |
| Rest | 0,5 Gew.-% |

Folgendes Ergebnis wurde erzielt:

| RZA [t/m$^3$/h] | 3,7 |
|---|---|
| Umsatz | 88,6 % |
| Selektivität | 98,3 % |
| B | 15,38 |
| 3-Methylbutanal | 7,7 Gew.-% |
| n-Pentanal | 1,4 Gew.- |
| Säure | 0,3 Gew.-% |
| 2-Propyl-5-methylhexenal | 12,6 Gew.-% |
| 2-Isopropylheptenal | 51,8 Gew.-% |
| 2-Propylheptenal | 22,4 Gew.-% |
| Aldoladditionsprodukte | 1,4 Gew.-% |
| Trimere | 1,8 Gew.-% |
| Rest | 0,7 Gew.-% |

**Beispiel 10:**

Dieses Beispiel beschreibt das erfindungsgemäße Verfahren für die Coaldolkondensation von n-Pentanal und 3-Methylbutanal (3-MBA) zu einem Isodecenal, bestehend aus den Isomeren 2-Propyl-5-methylhexenal und 2-Isopropylheptenal (1+3) sowie 2-Propylheptenal (1+1), unter Einsatz des Cosolvens Diethylenglykol (DEG). Der Reaktor (gemäß Beispiel 3) wurde mit einer Katalysatorbelastung von 400 kg/h bei einer Temperatur von 110 °C bei Eigendruck der Reaktionsteilnehmer durchströmt.

| Katalysator | |
|---|---|
| c NaOH | 3,52 Gew.-% |
| Diethylenglykol | 50,00 Gew.-% |
| Wasser | 46,48 Gew.-% |
| Eduktstrom [1/h] | 4,21 |

(fortgesetzt)

| Eduktzusammensetzung | |
|---|---|
| 3-Methylbutanal | 51,7 Gew.-% |
| n-Pentanal | 45,7 Gew.-% |
| Säure | 0,7 Gew.-% |
| Aldole | 1,0 Gew.-% |
| Trimere | 0,1 Gew.-% |
| Rest | 0,9 Gew.-% |

Folgendes Ergebnis wurde erzielt:

| | |
|---|---|
| RZA [t/m$^3$/h] | 4,1 |
| Umsatz | 97,5 % |
| Selektivität | 97,6 % |
| B | 14,09 |
| 3-Methylbutanal | 1,8 Gew.-% |
| n-Pentanal | 0,1 Gew.-% |
| Alkohol | 0,1 Gew.-% |
| Säure | 0,1 Gew.-% |
| 2-Propyl-5-methylhexenal | 20,5 Gew.-% |
| 2-Isopropylheptenal | 55,1 Gew.-% |
| 2-Propylheptenal | 18,1 Gew.-% |
| Aldoladditionsprodukte | 1,2 Gew.-% |
| Trimere | 2,1 Gew.-% |
| Rest | 0,9 Gew.-% |

**Beispiel 11:**

Dieses Beispiel beschreibt das erfindungsgemäße Verfahren für die Coaldolkondensation von 2-MBA und 3-MBA zu einem Isodecenal, bestehend aus den Isomeren 2-Isopropyl-4-methylhexenal (2+3) sowie 2-Isopropyl-5-methylhexenal (3+3). Der Reaktor (gemäß Beispiel 3) wurde mit einer Katalysatorbelastung von 400 kg/h bei einer Temperatur von 110 °C bei Eigendruck der Reaktionsteilnehmer durchströmt.

| Katalysator | |
|---|---|
| c NaOH | 2,51 Gew.-% |
| Wasser | 97,49 Gew.-% |
| Eduktstrom [1/h] | 4,39 |
| Eduktzusammensetzung | |
| 3-Methylbutanal | 48,0 Gew.-% |
| 2-Methylbutanal | 47,5 Gew.-% |
| Säure | 3,6 Gew.-% |
| Aldole | 0,1 Gew.-% |

(fortgesetzt)

| Eduktzusammensetzung | |
|---|---|
| Rest | 0,8 Gew.-% |

Folgendes Ergebnis wurde erzielt:

| RZA [t/m$^3$/h] | 1,4 |
|---|---|
| Umsatz | 35,7 % |
| Selektivität | 86,7 % |
| B | 15,59 |
| 3-Methylbutanal | 18,4 Gew.-% |
| 2-Methylbutanal | 39,4 Gew.-% |
| Alkohol | 0,4 Gew.-% |
| Säure | 2,8 Gew.-% |
| 2-Isopropyl-4-methylhexenal | 6,2 Gew.-% |
| 2-Isopropyl-5-methylhexenal | 28,0 Gew.-% |
| Aldoladditionsprodukte | 2,1 Gew.-% |
| Trimere | 1,5 Gew.-% |
| Rest | 1,2 Gew.-% |

**Beispiel 12:**

Dieses Beispiel beschreibt das erfindungsgemäße Verfahren für die Coaldolkondensation von 2-MBA und 3-MBA zu einem Isodecenal, bestehend aus den Isomeren 2-Isopropyl-4-methylhexenal (2+3) sowie 2-Isopropyl-5-methylhexenal (3+3), unter Einsatz des Cosolvens Diethylenglykol (DEG). Der Reaktor (gemäß Beispiel 3) wurde mit einer Katalysatorbelastung von 400 kg/h bei einer Temperatur von 110 °C bei Eigendruck der Reaktionsteilnehmer durchströmt.

| Katalysator | |
|---|---|
| c NaOH | 3,45 Gew.-% |
| Diethylenglykol | 50,00 Gew.-% |
| Wasser | 46,55 Gew.-% |
| Eduktstrom [l/h] | 4,14 |
| Eduktzusammensetzung | |
| 3-Methylbutanal | 47,6 Gew.-% |
| 2-Methylbutanal | 51,6 Gew.-% |
| Säure | 0,6 Gew.-% |
| Rest | 0,3 Gew.-% |

Folgendes Ergebnis wurde erzielt:

| RZA [t/m$^3$/h] | 2,4 |
|---|---|
| Umsatz | 63,1 % |
| Selektivität | 91,0 % |

(fortgesetzt)

| B | 14,04 |
|---|---|
| 3-Methylbutanal | 3,1 Gew.-% |
| 2-Methylbutanal | 28,7 Gew.-% |
| Alkohol | 1,5 Gew.-% |
| Säure | 1,2 Gew.-% |
| 2-Isopropyl-4-methylhexenal | 27,0 Gew.-% |
| 2-Isopropyl-5-methylhexenal | 34,0 Gew.-% |
| Aldoladditionsprodukte | 0,6 Gew.-% |
| Trimere | 2,5 Gew.-% |
| Rest | 1,4 Gew.-% |

**Beispiel 13:**

Dieses Beispiel beschreibt das erfindungsgemäße Verfahren für die Coaldolkondensation von n-Pentanal, 2-MBA und 3-MBA zu einem Isodecenal, bestehend aus den Isomeren 2-Propylheptenal (1+1), 2-Propyl-4-methylhexenal (1+2), 2-Isopropylheptenal (1+3), 2-Isopropyl-4-methylhexenal (2+3) sowie 2-Isopropyl-5-methylhexenal (3+3). Der Reaktor (gemäß Beispiel 3) wurde mit einer Katalysatorbelastung von 400 kg/h bei einer Temperatur von 110 °C bei Eigendruck der Reaktionsteilnehmer durchströmt.

| Katalysator | |
|---|---|
| c NaOH | 2,62 Gew.-% |
| Wasser | 97,38 Gew.-% |
| Eduktstrom [l/h] | 3,97 |
| Eduktzusammensetzung | |
| 3-Methylbutanal | 27,7 Gew.-% |
| 2-Methylbutanal | 35,3 Gew.-% |
| n-Pentanal | 34,8 Gew.-% |
| Säure | 0,4 Gew.-% |
| Aldole | 0,7 Gew.-% |
| Rest | 1,1 Gew.-% |

Folgendes Ergebnis wurde erzielt:

| RZA [t/m$^3$/h] | 2,5 |
|---|---|
| Umsatz | 62,8 % |
| Selektivität | 97,3 % |
| B | 15,19 |
| 3-Methylbutanal | 7,4 Gew.-% |
| 2-Methylbutanal | 22,5 Gew.-% |
| n-Pentanal nicht umgesetztes n-Pentanal | 1,6 Gew.-% |
| Alkohol | 0,2 Gew.-% |
| Säure | 0,4 Gew.-% |

(fortgesetzt)

| | |
|---|---|
| 2-Isopropyl-4-methylhexenal | 2,2 Gew.-% |
| 2-Isopropyl-5-methylhexenal | 7,3 Gew.-% |
| 2-Propyl-4-methylhexenal | 10,4 Gew.-% |
| 2-Isopropylheptenal | 31,1 Gew.-% |
| 2-Propylheptenal | 13,9 Gew.-% |
| Aldoladditionsprodukte | 1,7 Gew.-% |
| Trimere | 0,6 Gew.-% |
| Rest | 0,7 Gew.-% |

**Beispiel 14:**

Dieses Beispiel beschreibt das erfindungsgemäße Verfahren für die Coaldolkondensation von n-Pentanal, 2-MBA und 3-MBA zu einem Isodecenal bestehend aus den Isomeren 2-Propylheptenal (1+1), 2-Propyl-4-methylhexenal (1+2), 2-Isopropylheptenal (1+3), 2-Isopropyl-4-methylhexenal (2+3) sowie 2-Isopropyl-5-methylhexenal (3+3) unter Einsatz des Cosolvens Diethylenglykol (DEG). Der Reaktor (gemäß Beispiel 3) wurde mit einer Katalysatorbelastung von 400 kg/h bei einer Temperatur von 110 °C bei Eigendruck der Reaktionsteilnehmer durchströmt.

| | |
|---|---|
| Katalysator | |
| c NaOH | 3,50 Gew.-% |
| Diethylenglykol | 50,00 Gew.-% |
| Wasser | 46,50 Gew.-% |
| Eduktstrom [l/h] | 4,11 |
| 3-Methylbutanal | 33,0 Gew.-% |
| 2-Methylbutanal | 32,0 Gew.-% |
| n-Pentanal | 31,8 Gew.-% |
| Säure | 0,9 Gew.-% |
| Aldole | 1,0 Gew.-% |
| Trimere | 0,4 Gew.-% |
| Rest | 0, 9 Gew.-% |

Folgendes Ergebnis wurde erzielt:

| | |
|---|---|
| RZA [t/m$^3$/h] | 3,3 |
| Umsatz | 80,0 % |
| Selektivität | 98,1% |
| B | 14,01 |
| 3-Methylbutanal | 1,9 Gew.-% |
| 2-Methylbutanal | 13,9 Gew.-% |
| n-Pentanal (nicht umgesetztes n-Pentanal) | 0,1 Gew.-% |
| Alkohol | 1,1 Gew.-% |
| 2-Isopropyl-4-methylhexenal | 8,9 Gew.-% |
| 2-Isopropyl-5-methylhexenal | 12,9 Gew.-% |

(fortgesetzt)

| 2-Propyl-4-methylhexenal | 18,6 Gew.-% |
|---|---|
| 2-Isopropylheptenal | 29,4 Gew.-% |
| 2-Propylheptenal | 9,8 Gew.-% |
| Aldoladditionsprodukte | 0,8 Gew.-% |
| Trimere | 0,8 Gew.-% |
| Rest | 2,0 Gew.-% |

**Patentansprüche**

1. Verfahren zur katalytischen Aldolkondensation von Aldehyden durch Mehrphasenreaktion in einem Rohrreaktor **dadurch gekennzeichnet,** **daß** der Katalysator in der kontinuierlichen Phase und mindestens ein Aldehyd in einer dispergierten Phase enthalten ist und der Belastungsfaktor B des Rohrreaktors gleich oder größer 0,8 ist.

2. Verfahren nach Anspruch 1, **dadurch gekennzeichnet,** **daß** die Aldehyde 1 bis 15 Kohlenstoffatome enthalten.

3. Verfahren nach Anspruch 1 oder 2, **dadurch gekennzeichnet,** **daß** nur ein Aldehyd eingesetzt wird.

4. Verfahren nach Anspruch 1 oder 2, **dadurch gekennzeichnet,** **daß** ein Gemisch von mehreren Aldehyden mit der gleichen Anzahl von Kohlenstoffatomen eingesetzt werden

5. Verfahren nach Anspruch 1 oder 2, **dadurch gekennzeichnet,** **daß** ein Gemisch von mehreren Aldehyden mit unterschiedlicher Anzahl von Kohlenstoffatomen eingesetzt wird.

6. Verfahren nach einem der Ansprüche 1 bis 5, **dadurch gekennzeichnet,** **daß** die kontinuierliche Phase weitgehend aus Wasser besteht.

7. Verfahren nach einem der Ansprüche 1 bis 6, **dadurch gekennzeichnet,** **daß** die kontinuierliche Phase weitgehend aus Wasser und einem wasserlöslichen organischen Lösungsmittel besteht.

8. Verfahren nach einem der Ansprüche 1 bis 7, **dadurch gekennzeichnet,** **daß** als Katalysator eine wasserlösliche basische Verbindung eingesetzt wird.

9. Verfahren nach Anspruch 8, **dadurch gekennzeichnet,** **daß** als Katalysator Hydroxide, Hydrogencarbonate, Carbonate, Carboxylate oder ihre Gemische in Form ihrer Alkali- oder Erdalkaliverbindungen eingesetzt werden.

10. Verfahren nach einem der Ansprüche 1 bis 9, **dadurch gekennzeichnet,** **daß** der Katalysator in Konzentrationen von 0,1 bis 15 Massen-% in der kontinuierlichen Phase vorliegt.

**11.** Verfahren nach einem der Ansprüche 1 bis 10,
**dadurch gekennzeichnet,**
**daß** der Belastungsfaktor B größer oder gleich 0,9 ist.

**12.** Verfahren nach einem der Ansprüche 1 bis 11,
**dadurch gekennzeichnet,**
**daß** der Belastungsfaktor B größer oder gleich 1,0 ist.

**13.** Verfahren nach einem der Ansprüche 1 bis 12,
**dadurch gekennzeichnet,**
**daß** das Massenverhältnis der kontinuierlichen Phase zu der oder den dispergierten Phasen größer 2 ist.

**14.** Verfahren nach einem der Ansprüche 1 bis 13,
**dadurch gekennzeichnet,**
**daß** mindestens eine, einen Aldehyd enthaltende Phase durch die von der kontinuierlichen Phase in den Rohrreaktor eingebrachte Energie dispergiert wird.

**Claims**

**1.** Process for the catalytic aldol condensation of aldehydes by means of a multiphase reaction in a tube reactor, **characterized in that** the catalyst is present in the continuous phase and at least one aldehyde is present in a dispersed phase and the loading factor B of the tube reactor is equal to or greater than 0.8.

**2.** Process according to Claim 1, **characterized in that** the aldehydes contain from 1 to 15 carbon atoms.

**3.** Process according to Claim 1 or 2, **characterized in that** only one aldehyde is used.

**4.** Process according to Claim 1 or 2, **characterized in that** a mixture of two or more aldehydes having the same number of carbon atoms is used.

**5.** Process according to Claim 1 or 2, **characterized in that** a mixture of two or more aldehydes having a different number of carbon atoms is used.

**6.** Process according to any of Claims 1 to 5, **characterized in that** the continuous phase consists largely of water.

**7.** Process according to any of Claims 1 to 6, **characterized in that** the continuous phase consists largely of water and a water-soluble organic solvent.

**8.** Process according to any of Claims 1 to 7, **characterized in that** the catalyst used is a water-soluble basic compound.

**9.** Process according to Claim 8, **characterized in that** the catalyst used is a hydroxide, hydrogencarbonate, carbonate, carboxylate or a mixture thereof in the form of their alkali metal or alkaline earth metal compounds.

**10.** Process according to any of Claims 1 to 9, **characterized in that** the catalyst is present in the continuous phase in concentrations of from 0.1 to 15% by mass.

**11.** Process according to any of Claims 1 to 10, **characterized in that** the loading factor B is greater than or equal to 0.9.

**12.** Process according to any of Claims 1 to 11, **characterized in that** the loading factor B is greater than or equal to 1.0.

**13.** Process according to any of Claims 1 to 12, **characterized in that** the mass ratio of the continuous phase to the dispersed phase or phases is greater than 2.

**14.** Process according to any of Claims 1 to 13, **characterized in that** at least one phase containing an aldehyde is dispersed by the energy introduced into the tube reactor by means of the continuous phase.

**Revendications**

1. Procédé pour la condensation aldolique catalytique d'aldéhydes par une réaction en plusieurs phases dans un réacteur tubulaire, **caractérisé en ce que** le catalyseur est contenu dans la phase continue et au moins un aldéhyde est contenu dans une phase dispersée et le facteur de charge B du réacteur tubulaire est égal ou supérieur à 0,8.

2. Procédé selon la revendication 1, **caractérisé en ce que** les aldéhydes contiennent 1 à 15 atomes de carbone.

3. Procédé selon la revendication 1 ou 2, **caractérisé en ce qu'**on n'utilise qu'un aldéhyde.

4. Procédé selon la revendication 1 ou 2, **caractérisé en ce qu'**on utilise un mélange de plusieurs aldéhydes contenant le même nombre d'atomes de carbone.

5. Procédé selon la revendication 1 ou 2, **caractérisé en ce qu'**on utilise un mélange de plusieurs aldéhydes contenant un nombre différent d'atomes de carbone.

6. Procédé selon l'une quelconque des revendications 1 à 5, **caractérisé en ce que** la phase continue est constituée dans une large mesure d'eau.

7. Procédé selon l'une quelconque des revendications 1 à 6, **caractérisé en ce que** la phase continue est constituée dans une large mesure d'eau et d'un solvant organique soluble dans l'eau.

8. Procédé selon l'une quelconque des revendications 1 à 7, **caractérisé en ce qu'**on utilise comme catalyseur un composé basique soluble dans l'eau.

9. Procédé selon la revendication 8, **caractérisé en ce qu'**on utilise comme catalyseur des hydroxydes, des hydrogénocarbonates, des carbonates, des carboxylates ou leurs mélanges sous forme de leurs composés alcalins ou alcalino-terreux.

10. Procédé selon l'une quelconque des revendications 1 à 9, **caractérisé en ce que** le catalyseur se trouve en des concentrations de 0,1 à 15% en masse dans la phase continue.

11. Procédé selon l'une quelconque des revendications 1 à 10, **caractérisé en ce que** le facteur de charge B est supérieur ou égal à 0,9.

12. Procédé selon l'une quelconque des revendications 1 à 11, **caractérisé en ce que** le facteur de charge B est supérieur ou égal à 1,0.

13. Procédé selon l'une quelconque des revendications 1 à 12, **caractérisé en ce que** le rapport massique de la phase continue à la ou aux phase(s) dispersée(s) est supérieur à 2.

14. Procédé selon l'une quelconque des revendications 1 à 13, **caractérisé en ce qu'**au moins une phase, contenant un aldéhyde, est dispersée par l'énergie introduite par la phase continue dans le réacteur tubulaire.

Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1906850
- DE 927626
- GB 761203 A
- WO 9320034 A


**In der Beschreibung aufgeführte Nicht-Patentliteratur**

- *Hydrocarbon Processing,* Oktober 1980, vol. 2, 93-102
- **G. DÜMBGEN ; D. NEUBAUE R.** *Chemie-Ing.-Techn.,* 1969, vol. 41, 974
- VDI-Wärmeatlas 7. VDI-Verlag GmbH, 1994
- Grundlagen der Einphasen- und Mehrphasenströmungen. **HEINZ BRAUER.** Aarau und Frankfurt am Main. Verlag Sauerländer, 1971
- **S. ERGUN.** *Chem. Engng. Progr.,* 1948, vol. 48, 89
- **Y. SATO ; T.HIROSE ; F. TAKAHASHI ; M. TODA.** Pressure Loss and Liquid Hold Up in Packed Bed Reactor with Cocurrent Gas-Liquid Down Flow. *J. Chem. Eng. Of Japan,* 1973, vol. 6 (2), 147-152
- **D. SWEENEY.** A Correlation for Pressure Drop in Two-Phase Concurrent Flow in Packed Beds. *AIChE-Journal,* Juli 1967, vol. 13, 663-669
- **V. W. WEEKMAN ; J. E. MYERS.** Fluid-Flow Characteristics of Concurrent Gas-Liquid Flow in Packed Beds. *AIChE-Journal,* November 1964, vol. 10 (6), 951-957
- **R. P. LARKINS ; R. P. WHITE ; D. W. JEFFREY.** Two-Phase Concurrent Flow in Packed Beds. *AIChE-Journal,* Juni 1961, vol. 7 (2), 231-239
- **N. MIDOUX ; M. FAVIER ; J.- C. CHARPENTIER.** low Pattern, Pressure Loss and Liquid Holdup Data in Gas-Liquid Down-Flow Packed Beds with Foaming and Nonfoaming Liquids. *J. Chem. Eng. Of Japan,* 1976, vol. 9 (5), 350-356